# EUROPÄISCHE PATENTSCHRIFT

(11) **EP 3 510 015 B1**
(45) Veröffentlichungstag und Bekanntmachung des Hinweises auf die Patenterteilung: **21.10.2020**
(21) Anmeldenummer: 17772327.7
(22) Anmeldetag: 07.09.2017
(51) Int. Cl.: C07C 263/16, C07C 265/14, C07C 267/00, C08G 18/79, C08G 18/02

(54) **VERFAHREN ZUR HERSTELLUNG FLÜSSIGER, LAGERSTABILER CARBODIIMID- UND/ODER URETONIMIN-GRUPPEN AUFWEISENDER ORGANISCHER ISOCYANATE MIT NIEDRIGER FARBZAHL**
METHOD FOR PRODUCING LIQUID, STORAGE STABLE CARBODIIMIDE AND / OR URETONIMINE GROUPS CONTAINING ORGANIC ISOCYANATES WITH LOW COLOUR NUMBER
PROCÉDÉ DE FABRICATION DE CARBODIIMIDE LIQUIDE STABLE AU STOCKAGE ET/OU DE D'ISOCYANATES ORGANIQUES PRÉSENTANT DES GROUPES D'URÉTONIMINE À FAIBLE INDICE DE COLORATION

(30) Priorität: 08.09.2016 EP 16187725
(43) Veröffentlichungstag der Anmeldung: 17.07.2019
(73) Patentinhaber: Covestro Intellectual Property GmbH & Co. KG, 51373 Leverkusen (DE)
(72) Erfinder: ARNTZ, Hans-Detlef, 51491 Overath (DE); HAGEN, Torsten, 45257 Essen (DE); FELSKE, Ernst, 41464 Neuss (DE)
(74) Vertreter: Levpat
(86) Internationale Anmeldenummer: PCT/EP2017/072517
(87) Internationale Veröffentlichungsnummer: WO 2018/046622

(56) Entgegenhaltungen:
- EP-A2- 0 515 933
- EP-A2- 1 671 988
- DE-A1- 2 537 685

## Beschreibung

Die Erfindung betrifft ein Verfahren zur Herstellung flüssiger, lagerstabiler Carbodiimid- (CD) und/oder Uretonimin- (UI) Gruppen aufweisender Isocyanatmischungen bzw. davon abgeleiteter Präpolymere, wobei
(i) in einem ersten Schritt ein Ausgangsisocyanat, das ein organisches Isocyanat oder ein Gemisch mehrerer organischer Isocyanate ist, mit einem Katalysator K der Formel *cyclo*-C₄H₆P(O)R, wobei der Substituent R für einen gesättigten oder ungesättigten, optional substituierten, insbesondere Halogen-substituierten, organischen Rest, steht, teilweise carbodiimidisiert wird und anschließend
(ii) in einem zweiten Schritt die Carbodiimidisierungsreaktion bei einer Temperatur ≤ 80 °C durch Zugabe eines Stoppers abgestoppt wird,
wobei als Stopper ein organisches Silan S der allgemeinen Formel HₙSiX₄₋ₙ, wobei X für einen gesättigten oder ungesättigten, optional substituierten, insbesondere Halogen-substituierten, organischen Rest, steht, eingesetzt wird.

CD- und /oder UI-Gruppen aufweisende Isocyanatmischungen können in einfacher Weise mit den hoch wirksamen Katalysatoren aus der Phospholin-Reihe, insbesondere der Phospholinoxid-Reihe, nach den Verfahren gemäß US-A-2,853,473, US-A-6,120,699 und EP-A-0 515 933 hergestellt werden.

Die hohe katalytische Aktivität der Phospholinkatalysatoren, insbesondere der Phospholinoxidkatalysatoren, ist einerseits erwünscht, um die Carbodiimidisierungsreaktion unter schonenden Temperaturbedingungen anzustoßen, andererseits ist die wirksame Abstoppung der Phospholin-Katalyse bzw. der Phospholinoxid-Katalyse bis heute nicht ohne Herausforderungen. Die carbodiimidisierten Isocyanate neigen zur Nachreaktion, d. h. sie gasen infolge CO₂-Entwicklung aus. Dies führt dann besonders bei höheren Temperaturen zu einem Druckaufbau beispielsweise in den Lagerbehältern. Außerdem kann die Gewinnung von farbhellen Produkten und insbesondere die Farbstabilität nach längerer Lagerung immer wieder problematisch sein.

Es hat nicht an Versuchen gefehlt, eine wirksame Abstoppung der Phospholin-Katalyse zu finden. Derartige Stopper sind z. B. in den Patentanmeldungen DE-A-25 37 685, EP-A-0 515 933, EP-A-0 609 698, EP-A-1 607 425, US-A-6,120,699 und US 2007/213496 erwähnt und umfassen z. B. Säuren, Säurechloride, Chloroformiate, silylierte Säuren und Halogenide der Hauptgruppenelemente. Im Stand der Technik offenbarte Stopper sind insbesondere Ehyltrifluoromethylsulfonat (ETF), Trimethylsilyltrifluoromethansulfonat (TMST) als Methylierungs- bzw. Silylierungsreagenz oder starke Säuren wie Trifluoromethansulfonsäure (TFMSA) oder Perchlorsäure.

Nach der Lehre der EP-A-0 515 933 werden mittels Phospholin-Katalyse hergestellte CD/UIhaltige Isocyanatmischungen mit mindestens der äquimolaren Menge, bevorzugt der 1- bis 2-fachen molaren Menge, bezogen auf den eingesetzten Katalysator, an einer silylierten Säure (bzw. deren Ester) wie z. B. Trimethylsilyltrifluormethansulfonat (TMST) abgestoppt. In der Praxis hat sich jedoch erwiesen, dass solchermaßen hergestellte CD/UI-enthaltende Isocyanate zur Herstellung von Prepoymeren, d. h. Umsetzungsprodukten von diesen CD/UI-enthaltenden Isocyanaten mit Polyolen, nur bedingt geeignet sind. Die entsprechend hergestellten Umsetzungsprodukte aus Polyolen und den CD/UI-modifizierten Isocyanaten neigen zum Ausgasen, was zu einem Druckaufbau in den Transportbehältern oder zum Schäumen bei der Handhabung derartiger Produkte führen kann.

Man kann dieses Problem dadurch umgehen, dass man die zum Abstoppen des Phospholin-Katalysators benutzte silylierte Säure analog der EP-A-0 515 933 in höheren molaren Äquivalenten (z. B. 5 : 1 bis 10 : 1, bezogen auf den Katalysator) einsetzt. In der Praxis zeigt sich dann jedoch, dass die erhaltenen CD/UI modifizierten Isocyanate eine deutlich schlechtere Farbzahl aufweisen. Dies gilt dann auch für die hieraus hergestellten Präpolymere.

Dies gilt auch, wenn der Phospholinkatalysator mit Säuren vom Typ der Trifluormethansulfonsäure entsprechend der US-A-6,120,699 abgestoppt wird. Auch daraus hergestellte Präpolymere weisen eine erheblich erhöhte Farbzahl auf.

Gemäß der Lehre der EP-A-1 616 858 wird zusätzlich zu der in EP-A-0 515 933 beschriebenen silylierten Säure eine nicht silylierte Säure und/oder ein Säurechlorid und/oder ein Sulfonsäueester eingesetzt. Aber auch dies vermag die geschilderten Probleme nicht völlig zu lösen, denn bei Temperaturbelastung während der Lagerung oder bei der Weiterverarbeitung zu (Semi)-Präpolymeren kann der Katalysator wieder aktiv werden.

Gemäß der Lehre der EP-A-1 671 988 wird als Stopper ein Alkylierungsmittel, bevorzugt ein Ester der Trifluormethansulfonsäure, eingesetzt. Dieses Verfahren hat jedoch den Nachteil, dass die Kondensationsreaktion bei Zusatz von Polyolen, wie sie bei der Präpolymerherstellung eingesetzt werden, wieder anspringen kann, was zu niedrigeren als den erwarteten Isocyanatgehalten führt.

WO 2007/076998 lehrt die Durchführung der Carbodiimidisierung in Gegenwart eines sekundären oder tertiären Amins und Abstoppen der Reaktion mit den bekannten Reagenzien des Standes der Technik. Diese Zusätze dienen zur Bindung der im Isocyanat herstellungsbedingt in Spuren enthaltenen Salzsäure, die die Carbodiimidbildung beeinträchtigt, und erhöhen nur indirekt die Stabilität des Produkts, weil die Katalysatorkonzentration geringfügig gesenkt werden kann, ohne die Reaktionszeiten zu verlängern.

US 2007/213496 offenbart die Herstellung Carbodiimid-haltiger Polyisocyanate unter Katalyse von Phospholinoxid-Derivaten bei moderaten Reaktionstemperaturen von 80 °C bis 130 °C. Das Abstoppen der Reaktion erfolgt in zwei Stufen mit sukzessive sinkender Temperatur. In der ersten Stufe wird bevorzugt eine Säure, ein Peroxid oder Säurechlorid als Stopper eingesetzt.

Allen beschriebenen Verfahren des Standes der Technik ist gemein, dass sich die Stabilität der so hergestellten Carbodiimid-haltigen Polyisocyanate bei längerer Lagerung und/oder unter Temperaturbelastung und/oder bei pH-Wert-Änderung (die beiden letztgenannten Aspekte sind insbesondere für Weiterverarbeitung zu Präpolymeren bedeutsam) häufig als unzureichend darstellt. Dies kann Farbverschlechterungen sowie ein erneutes Einsetzen der unter CO₂-Abspaltung ablaufenden Kondensationsreaktion zum Carbodiimid zur Folge haben. Selbst ohne längere Lagerung ist die Farbe von Carbodiimid-haltigen Polyisocyanaten nicht immer zufriedenstellend.

Weitere Verbesserungen auf diesem Gebiet der Technik wären daher wünschenswert. Insbesondere wäre es wünschenswert, ein Verfahren zur Herstellung flüssiger, Carbodiimid- und/oder Uretonimingruppen aufweisender Isocyanatmischungen zur Verfügung zu haben, welches die oben angesprochenen Mängel nicht aufweist. Die herzustellenden flüssigen, Carbodiimid- und/oder Uretonimingruppen aufweisenden Isocyanatmischungen sollten insbesondere niedrige Farbzahlen aufweisen und lagerstabil sein, d. h. Farbwert, NCO-Gehalt und Viskosität sollten durch Lagerung möglichst wenig verändert werden. Insbesondere wünschenswert wäre es auch, wenn die so hergestellten flüssigen, Carbodiimid- und/oder Uretonimingruppen aufweisenden Isocyanatmischungen zu Präpolymeren niedriger Farbzahl und hoher Lagerstabilität umgesetzt werden könnten.

Dem vorstehend Gesagten Rechnung tragend betrifft die Erfindung ein Verfahren zur Herstellung Carbodiimid- und/oder Uretonimingruppen aufweisender organischer Isocyanate, bei dem
(i) in einem ersten Schritt ein Ausgangsisocyanat, das ein organisches Isocyanat oder ein Gemisch mehrerer organischer Isocyanate ist, mit einem Katalysator K vom Phospholinoxid-Typ teilweise carbodiimidisiert wird und anschließend
(ii) in einem zweiten Schritt die Carbodiimidisierungsreaktion bei einer Temperatur ≤ 80 °C durch Zugabe eines Stoppers abgestoppt wird,
wobei als Stopper ein organisches Silan S der allgemeinen Formel HₙSiX₄₋ₙ, wobei n eine natürliche Zahl im Bereich von 1 bis 3 ist, wobei X für einen gesättigten oder ungesättigten, optional substituierten, insbesondere Halogen-substituierten, organischen Rest, insbesondere Methyl oder Phenyl, besonders bevorzugt für Phenyl (C₆H₅) steht, eingesetzt wird. Insbesondere bevorzugt gilt: X = Phenyl und n = 2 oder 3.

Es folgt zunächst eine Kurzzusammenfassung verschiedener möglicher **Ausführungsformen der Erfindung:**
In einer **ersten Ausführungsform** der Erfindung, die mit allen anderen Ausführungsformen kombiniert werden kann, wird als Ausgangsisocyanat ein solches mit einer APHA-Hazen-Farbzahl von ≤ 100, bevorzugt ≤ 50, eingesetzt.

In einer **zweiten Ausführungsform** der Erfindung, die mit allen anderen Ausführungsformen kombiniert werden kann, sofern diese nicht den Einsatz anderer Ausgangsisocyanate als der Folgenden vorsehen, wird das Ausgangsisocyanat ausgewählt aus der Gruppe bestehend aus
- Toluylendisocyanat (TDI) und
- Methylendiphenyldiisocyanat als Isomerengemisch in beliebigen Anteilen der 2,2'-, 2,4'- und 4,4'-Isomeren.

In einer **dritten Ausführungsform** der Erfindung, die mit allen anderen Ausführungsformen kombiniert werden kann, sofern diese nicht den Einsatz anderer Ausgangsisocyanate als der Folgenden vorsehen, wird das Ausgangsisocyanat ausgewählt aus der Gruppe bestehend aus
- 4,4'-Methylendiphenyldiisocyanat enthaltend bis zu 4,0 Massen-% 2,2'-Methylendiphenyldiisocyanat, bezogen auf die Gesamtmasse aller Methylendiphenyldiisocyanat-Isomeren und
- Gemischen aus 2,4'-Methylendiphenyldiisocyanat und 4,4'-Methylendiphenyldiisocyanat enthaltend bis zu 4,0 Massen-% 2,2'-Methylendiphenyldiisocyanat, bezogen auf die Gesamtmasse aller Methylendiphenyldiisocyanat-Isomeren.

In einer **vierten Ausführungsform** der Erfindung, die mit allen anderen Ausführungsformen kombiniert werden kann, sofern diese nicht den Einsatz anderer Ausgangsisocyanate als das nachfolgend definierte vorsehen, enthält das Ausgangsisocyanat mindestens 99,0 Massen-%, bevorzugt 99,5 Massen-%, bezogen auf die Gesamtmasse des Ausgangsisocyanats, an Methylendiphenyldiisocyanat-Isomeren, wobei der Anteil des 4,4'-Methylendiphenyldiisocyanats, bezogen auf die Gesamtheit aller Methylendiphenyldiisocyanat-Isomeren im Ausgangsisocyanat, mindestens 97,0 %, bevorzugt mindestens 98,0 % beträgt.

In einer **fünften Ausführungsform** der Erfindung, die mit allen anderen Ausführungsformen kombiniert werden kann, sofern diese nicht den Einsatz anderer Ausgangsisocyanate als das nachfolgend definierte vorsehen, wird als Ausgangsisocyanat ein Gemisch aus Methylendiphenyldiisocyanat-Isomeren und Polymethylenpolyphenylenpolyisocyanat mit einem Gehalt an Methylendiphenyldiisocyanat-Isomeren von insgesamt 15 Massen-% bis < 95 Massen-%, bezogen auf die Gesamtmasse von Methylendiphenyldiisocyanat-Isomeren und Polymethylenpolyphenylenpolyisocyanat, eingesetzt.

In einer **sechsten Ausführungsform** der Erfindung, die mit allen anderen Ausführungsformen kombiniert werden kann, gilt:
X = Phenyl und n = 2 oder 3.

In einer **siebten Ausführungsform** der Erfindung, die mit allen anderen Ausführungsformen kombiniert werden kann, wird als Katalysator K vom Phospholinoxid-Typ eine der folgenden Verbindungen oder ein Gemisch aus beiden eingesetzt:

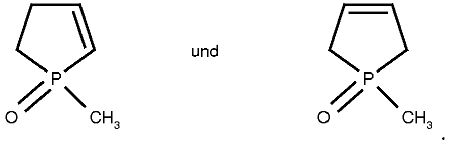

In einer **achten Ausführungsform** der Erfindung, die mit allen anderen Ausführungsformen kombiniert werden kann, wird die Carbodiimidisierungsreaktion in Schritt (i) im Temperaturbereich von 50 °C bis 150 °C, vorzugsweise von 60 °C bis 100 °C, durchgeführt.

In einer **neunten Ausführungsform** der Erfindung, die mit allen anderen Ausführungsformen kombiniert werden kann, wird die Carbodiimidisierungsreaktion aus Schritt (i) bei Erreichen eines Carbodiimidisierungsgrades im Bereich von 3,0 % bis 50 % gemäß Schritte (ii) abgestoppt.

In einer **zehnten Ausführungsform** der Erfindung, die mit allen anderen Ausführungsformen kombiniert werden kann, wird ein Stoffmengenverhältnis n(S)/n(K) von organischem Silan S zu Katalysator K im Bereich von 1 bis 20 verwendet.

In einer **elften Ausführungsform** der Erfindung, die mit allen anderen Ausführungsformen kombiniert werden kann, wird die Reaktionsmischung aus Schritt (i) vor der Durchführung von Schritt (ii) auf eine Temperatur im Bereich von 50 °C bis 70 °C abgekühlt.

In einer **zwölften Ausführungsform** der Erfindung, die mit allen anderen Ausführungsformen außer der im Folgenden beschriebenen dreizehnten Ausführungsform kombiniert werden kann, wird in Schritt (ii) neben dem Silan S kein weiterer Stopper eingesetzt.

In einer **dreizehnten Ausführungsform** der Erfindung, die mit allen anderen Ausführungsformen außer der zwölften Ausführungsform kombiniert werden kann, wird in Schritt (ii) zusätzlich zum Silan S ein weiterer Stopper ausgewählt aus der Gruppe bestehend aus Säuren, Säurechloriden, Sulfonsäureestern und Gemischen aus den vorgenannten Stoffen eingesetzt.

In einer **vierzehnten Ausführungsform** der Erfindung, die mit allen anderen Ausführungsformen kombiniert werden kann, wird das in Schritt (ii) erhaltene Carbodiimid- und/oder Uretonimingruppen aufweisende organische Isocyanat mit einem Polyol zu einem Präpolymer umgesetzt wird.

Die zuvor kurz geschilderten Ausführungsformen und weitere mögliche Ausgestaltungen der Erfindung werden im Folgenden näher erläutert. Verschiedene Ausführungsformen sind, sofern sich aus dem Kontext für den Fachmann nicht eindeutig das Gegenteil ergibt, beliebig miteinander kombinierbar.

Als **Ausgangsisocyanate** für das erfindungsgemäße Verfahren können beliebige organische Isocyanate, insbesondere solche mit einer APHA-Hazen-Farbzahl (Platin-Cobalt-Farbzahl) von ≤ 100, bevorzugt ≤ 50, eingesetzt werden. Maßgeblich für die Bestimmung der Hazen-Farbzahl ist dabei die Methode DIN EN ISO 6271-1:2005-03) in Substanz gegen eine salzsaure wässrige Kaliumhexachloroplatinat(IV)-/Kobalt(II)-chlorid Lösung als Referenz bei einer Schichtdicke von 5 cm. Als Messgerät kann z. B. ein Photometer LICO® 100 der Fa. Dr. Lange eingesetzt werden.

Vorzugsweise wird das erfindungsgemäße Verfahren zur Carbodiimidisierung von organischen Di- und Polyisocyanaten verwendet, die insbesondere in der Polyurethan-Chemie eingesetzt werden. Geeignet sind insbesondere:
- Aromatische Diisocyanate wie **(i)** Toluylendisocyanat (TDI), insbesondere Gemische von 2,4- und 2,6-TDI (*"meta-TDI"*), (**ii**) Methylendiphenyldiisocyanat als Isomerengemisch in beliebigen Anteilen der 2,2'-, 2,4'- und 4,4'-Isomeren, insbesondere 4,4'-Methylendiphenyldiisocyanat sowie Gemische aus 2,4'-Methylendiphenyldiisocyanat und 4,4'-Methylendiphenyldiisocyanat, wobei in beiden letztgenannten Stoffgruppen jeweils bis zu 4,0 Massen-% des 2,2'-Isomeren, bezogen auf die jeweilige Gesamtmasse an Methylendiphenyldiisocyanat-Isomeren, enthalten sein können;
- Gemische aus Methylendiphenyldiisocyanat-Isomeren und Polymethylenpolyphenylenpolyisocyanat mit einem Gehalt an Methylendiphenyldiisocyanat-Isomeren von insgesamt 15 Massen-% bis < 95 Massen-%, bezogen auf die Gesamtmasse von Methylendiphenyldiisocyanat-Isomeren und Polymethylenpolyphenylenpolyisocyanat.

Besonders bevorzugt enthält das Ausgangsisocyanat mindestens 99,0 Massen-%, bevorzugt 99,5 Massen-%, bezogen auf die Gesamtmasse des Ausgangsisocyanats, an Methylendiphenyldiisocyanat-Isomeren, wobei der Anteil des 4,4'-Methylendiphenyldiisocyanats, bezogen auf die Gesamtheit aller Methylendiphenyldiisocyanat-Isomeren im Ausgangsisocyanat, mindestens 97,0 %, bevorzugt mindestens 98,0 % beträgt (aufgrund der identischen molaren Masse der verschiedenen Methylendiphenyldiisocyanat-Isomeren spielt es keine Rolle, ob Massen-% oder Mol-% zugrunde gelegt werden).

Die Herstellung solcher organischer Isocyanate ist im Stand der Technik hinlänglich bekannt und wird daher an dieser Stelle nicht näher beschrieben.

**Schritt (i)** des erfindungsgemäßen Verfahrens wird in Gegenwart von Katalysatoren K vom Phospholinoxid-Typ durchgeführt. Unter Katalysatoren vom Phospholinoxid-Typ werden im Rahmen der vorliegenden Erfindung am Phosphoratom substituierte 1-Oxo-Phosphacyclopentene, *cyclo*-C₄H₆P(O)R, verstanden, wobei der Substituent R für einen gesättigten oder ungesättigten, optional substituierten, insbesondere Halogen-substituierten, organischen Rest, insbesondere Methyl oder Phenyl, steht. Die Katalysatoren vom Phospholinoxid-Typ sind beispielsweise aus EP-A-0 515 933 und US-A-6,120,699 bekannt. Typische Beispiele dieser Katalysatoren sind insbesondere die aus dem Stand der Technik bekannten Gemische der Phospholinoxide der Formel:

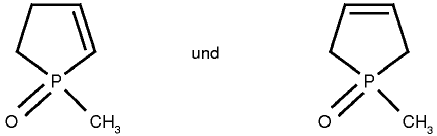

Die Menge des eingesetzten Katalysators hängt von der Qualität der Ausgangsisocyanate ab. Die jeweils notwendige Katalysatorenmenge lässt sich daher am einfachsten in einem Vorversuch bestimmen.

Die Carbodiimidisierungsreaktion in Schritt (i) wird bevorzugt im Temperaturbereich von 50 °C bis 150 °C, vorzugsweise von 60 °C bis 100 °C, durchgeführt. Die optimale Reaktionstemperatur richtet sich nach der Art der Ausgangsisocyanate und/oder des eingesetzten Katalysators und kann in einem einfachen Vorversuch ermittelt werden.

Die Carbodiimidisierungsreaktion wird bevorzugt bei Erreichen eines Carbodiimidisierungsgrades von 3,0 % bis 50 %, vorzugsweise 5,0 % bis 30 %, abgebrochen. Unter dem *Carbodiimidisierungsgrad* wird dabei der Prozentsatz der carbodiimidisierten Isocyanatgruppen bezogen auf die Gesamtmenge der im **Ausgangs**isocyanat vorliegenden Isocyanatgruppen verstanden. Der Carbodiimidisierungsgrad kann während der Durchführung des erfindungsgemäßen Verfahrens durch Bestimmung des Isocyanatgehalts z. B. mittels dem Fachmann an sich bekannter Titration (EN ISO 11909:2007, Anhang A) oder mittels online-Verfahren bestimmt werden. Ein geeignetes online-Verfahren ist z. B. die Nahinfrarot- oder die Mittelinfrarot-Analytik. Der Carbodiimidisierungsgrad kann während der Durchführung des erfindungsgemäßen Verfahrens ebenfalls z. B. an der Menge des im Reaktorgemisch entweichenden Kondensationsprodukts Kohlendioxid erkannt werden (bei der Kondensation von 2 Molen NCO-Gruppen entsteht 1 Mol CO₂). Diese volumetrisch mittels eines Gaszählers bestimmbare Kohlendioxidmenge gibt somit zu jedem Zeitpunkt Auskunft über den erreichten Carbodiimidisierungsgrad. Darüber hinaus können grundsätzlich auch andere geeignete, dem Fachmann bekannte offline- oder online-Methoden der Prozessverfolgung eingesetzt werden. Grundsätzlich ergeben alle diese Messmethoden im Rahmen üblicher, für die Zwecke der Erfindung insignifikanter, Fehlerbreiten die gleichen Ergebnisse. Sollte dies im Einzelfall wider Erwarten nicht der Fall sein, so ist der aus der per volumetrischer Messung (wie zuvor beschrieben) der Menge an entweichendem Kohlendioxid und dem per Titration gemäß EN ISO 11909:2007 bestimmten Isocyanat-Gehalt des Ausgangsisocyanats bestimmte Carbodiimidisierungsgrad maßgeblich. In den im Beispielteil beschriebenen Versuchen wurde der Volumenstrom des Kondensationsprodukts CO₂ kontinuierlich mit einem Gaszähler (einer sog. "Gasuhr") verfolgt.

Zum Beenden der Carbodiimidisierungsreaktion in **Schritt (ii)** wird als Stopper ein organisches Silan der allgemeinen Formel HₙSiX₄₋ₙ, wobei n eine natürliche Zahl im Bereich von 1 bis 3 ist, X für einen gesättigten oder ungesättigten, optional substituierten, insbesondere Halogen-substituierten, organischen Rest, insbesondere Methyl oder Phenyl, besonders bevorzugt für Phenyl (C₆H₅) steht, eingesetzt. Es ist auch möglich, eine Mischung verschiedener Silane der genannten Art einzusetzen. Der zusätzliche Einsatz anderer Stopper ist nicht erforderlich und wird daher auch in einer bevorzugten Ausgestaltung der Erfindung nicht durchgeführt. Besonders bevorzugt ist X = C₆H₅ und - infolge des im Vergleich zum Triphenylsilan geringeren Schmelzpunktes (und damit einhergehend leichterer Verarbeitbarkeit) von Mono- bzw. Diphenylsilan - n = 2 oder 3, insbesondere n = 2.

Erfindungsgemäß einsetzbare organische Silane sind kommerziell erhältlich. Der Einsatz von Phenylsilan in der Synthese von 2-Phosphabicyclo[3.3.0]-octen-Ringsystemen ist in Tetrahedron Letters 44 (2003), 5469 - 5472 (Autoren: Z. Pakulski, R. Kwiatosz und K. M. Pietrusiewicz) beschrieben.

Das organische Silan wird bevorzugt in mindestens äquimolarer Menge bezogen auf die Stoffmenge des eingesetzten Katalysators vom Phospholinoxid-Typ eingesetzt. Insbesondere wird bevorzugt ein bis zu 20-facher, besonders bevorzugt bis zu 10-facher, molarer Überschuss des organischen Silans bezogen auf die Stoffmenge des eingesetzten Katalysators vom Phospholinoxid-Typ verwendet.

Vor der Zugabe des organischen Silans wird die in Schritt (i) erhaltene, das Carbodiimid- und/oder Uretonimingruppen aufweisende organische Isocyanat enthaltende, Reaktionsmischung erforderlichenfalls auf eine Temperatur ≤ 80 °C, bevorzugt auf eine Temperatur im Bereich von 50 °C bis 70 °C abgekühlt. Sollte die Umsetzung in Schritt (i) bei einer Temperatur ≤ 80 °C, insbesondere bei einer Temperatur im Bereich von 50 °C bis 70 °C, durchgeführt werden, ist eine weitere Abkühlung überflüssig, und der Stopper wird im Anschluss an Schritt (i) unmittelbar zugegeben.

Alternativ zur alleinigen Verwendung von Silanen als Abstopper kann in einer anderen bevorzugten Ausgestaltung des erfindungsgemäßen Verfahren eine Kombination eines Silans mit einer Säure und/oder einem Säurechlorid und/oder einem Sulfonsäureester als zusätzlichem Stabilisator (der ebenfalls als Stopper wirkt) eingesetzt werden. Durch die Mitverwendung eines zusätzlichen Stabilisators kann die Einsatzmenge des Silans reduziert werden. Die Zugabe dieses Stabilisators kann entweder zeitgleich mit der Zugabe des Abstoppers oder in einem nachgeschalteten Schritt erfolgen.

Als Säuren können ggf. halogenierte, aliphatische und/oder cycloalophatische und/oder aromatische Mono-, Di- und/oder Poly-Carbonsäuren wie z.B. Essigsäure, Adipinsäure, Cyclohexandicarbonsäure, α-Chlorpropionsäure, Benzoesäure, Phthalsäure, Isophtalsäure usw., sowie Sulfonsäuren, HCl, Schwefelsäure und/oder Phosphorsäure bzw. deren Mono- und/oder Diester, wie z.B. Dibutylphosphat, eingesetzt werden. Als Säurechloride können die aus den ggf. halogenierten, aliphatischen und/oder cycloalophatischen und/oder aromatischen Mono-, Di- und/oder Poly-Carbonsäuren bzw. Sulfonsäuren abgeleiteten Säurechloride sowie Carbamidsäurechloride, wie z.B. n-Butylcarbamidsäurechlorid, eingesetzt werden. Als Sulfonsäureester können z.B. p-Toluolsulfonsäuremethylester, p-Toluolsulfonsäureethylester eingesetzt werden.

Die zusätzlichen Stabilisatoren werden dabei in Mengen von insgesamt zwischen 10 ppm und 1000 ppm (1 ppm: 1 Massenteil auf 1000000 Massenteile), bevorzugt zwischen 10 ppm und 500 ppm, besonders bevorzugt zwischen 50 ppm und 250 ppm, bezogen auf die Masse des Ausgangsisocyanats, zugesetzt.

Der Einsatz von Farbstabilisatoren, wie sie üblicherweise Isocyanaten zugesetzt werden, ist auch im erfindungsgemäßen Verfahren möglich. Die Farbstabilisatoren können bevorzugt dem Reaktionsprodukt nach vollendeter Umsetzung zugesetzt werden. Derartige Stabilisatoren sind generell dem Fachmann bekannt und umfassen z. B. Substanzen aus der Gruppe der sterisch gehinderten Phenole, der Phosphorigsäureester oder der tertiären Amine. Die Farbstabilisatoren können jeweils für sich allein oder im Gemisch mit anderen Vertretern der gleichen oder verschiedener Substanzgruppen eingesetzt werden. Die Mengen der eingesetzten Farbstabilisatoren bewegen sich in der dem Fachmann bekannten Größenordnung, üblicherweise im Bereich von 100 ppm bis 10000 ppm für die Einzelsubstanz bzw. das Gemisch, bezogen auf das als Ausgangsmaterial verwendete Isocyanat bzw. das Reaktionsprodukt der Carbodiimidisierung.

Es hat sich gezeigt, dass durch die Zugabe der organischen Silane allein oder in Kombination mit einer Säure und/oder einem Säurechlorid und/oder einem Sulfonsäureester als zusätzlichem Stabilisator nach dem erfindungsgemäßen Verfahren sowohl die Phospholinoxid-Katalyse wirksam abgestoppt werden kann, als auch gleichzeitig niedrige Farbwerte im erzeugten Carbodiimid-modifizierten Isocyanat und daraus hergestellten Präpolymeren erhalten werden können. Bei Einsatz der silylierten Säure als Abstopper gemäß dem Stand der Technik (EP-A-0 515 933) lässt sich die Phospholinoxid-Katalyse nur durch Zugabe großer Mengen an silylierter Säure wirksam abstoppen, was aber zu erhöhten Farbwerten der so erzeugten Isocyanatmischungen und daraus hergestellten Präpolymeren führt.

Isocyanatgruppen enthaltende **Präpolymere** werden durch Umsetzung der nach dem erfindungsgemäßen Verfahren hergestellten Carbodiimid- und/oder Uretonimingruppen aufweisenden organischen Isocyanate mit in der Polyurethanchemie üblichen Polyolen erhalten. Geeignete Polyole sind einerseits einfache mehrwertige Alkohole einer Molekülmasse im Bereich von 62 g/mol bis 599 g/mol, vorzugsweise von 62 g/mol bis 300 g/mol, wie insbesondere Ethylenglykol, Trimethylolpropan, Propandiol-1,2, Butandiol-1,2, Butandiol-2,3, Butandiol-1,3, Butandiol-1,4, Hexandiol, Octandiol, Dodecandiol und/oder Octadecandiol. Besonders bevorzugt aus der Gruppe der einfachen mehrwertigen Alkohole sind Propandiol-1,2, Butandiol-1,3, Butandiol-1,4 oder Mischungen dieser Alkohole.

Geeignete (und stärker bevorzugte) Polyole sind andererseits höhermolekulare Polyetherpolyole und/oder Polyesterpolyole der aus der Polyurethanchemie an sich bekannten Art mit zahlengemittelten mittleren Molmassen von 600 g/mol bis 8000 g/mol, vorzugsweise 800 g/mol bis 4000 g/mol, die mindestens zwei, insbesondere 2 bis 8, vorzugsweise 2 bis 4 primäre und/oder sekundäre Hydroxylgruppen aufweisen. Beispiele derartiger Polyole sind in der US-Patentschrift 4,218,543, Spalte 7, Zeile 29 bis Spalte 9, Zeile 32 beschrieben.

Die Vorteile des erfindungsgemäßen Verfahrens sind augenscheinlich: Sowohl die Carbodiimid und/oder Uretonimingruppen haltigen Isocyanate als auch die daraus hergestellten Präpolymere weisen eine gute Lagerstabilität und eine helle Farbe auf.

Diese Carbodiimid- und/oder Uretonimingruppen aufweisenden organischen Isocyanate und die daraus durch Umsetzung mit Polyolen hergestellten Präpolymere stellen wertvolle Ausgangsmaterialien zur Herstellung von Polyurethankunststoffen durch Umsetzung mit Polyolen (z. B. mit Polyetherpolyolen oder Polyesterpolyolen) nach dem Isocyanat-Polyadditionsverfahren dar.

### Beispiele

**Einsatzstoffe:**

| | |
|---|---|
| Isocyanat: | Desmodur 44M®, Covestro Deutschland AG (4,4'-Methylendiphenyldiisocyanat, NCO-Gehalt: 33,6 Massen-%); |
| Katalysator: | Phospholinoxid-Typ: technisches Gemisch aus 1-Methyl-1-oxo-1-phosphacyclopent-2-en und 1-Methyl-1-oxo-1-phosphacyclopent-3-en in Toluol, (Massenanteil 1,0 %); |
| Antioxidant: | Octadecyl 3-(3,5-di-tert-butyl-4-hydroxyphenyl)propionat (Irganox 1076 Ciba®); |
| Stopper: | Trifluormethansulfonsäureethylester (TFMSEE), Phenylsilan, Diphenylsilan, Triphenylsilan (alle Aldrich) oder Baysilon MH15 ("Momentive", Covestro Deutschland AG, *Polymethylhydrogensiloxan),* |
| Polyol: | linearer auf Propylenglykol gestarteter Propylenoxid-Polyether einer Hydroxylzahl von 515 mg KOH/g und einer Viskosität von 55 mPas bei 23 °C. |

**Analytik:**

| | |
|---|---|
| Isocyanatgehalt: | EN ISO 11909:2007, Anhang A; |
| Viskosität: | DIN 53019-1:2008-09; Rotationsviskosimeter Anton Paar; |
| Hazen-Farbzahl (=APHA-Hazen-Farbzahl, Platin-Cobalt-Farbzahl): | DIN EN ISO 6271-1:2005-03. |

### I. Allgemeine Vorschrift zur Herstellung des Carbodiimid- und/oder Uretonimingruppen aufweisenden organischen Isocyanats und zur Durchführung von Lagertests:

1. 1,000 kg Desmodur 44M® mit einer Hazen-Farbzahl von < 15 APHA werden in einer Stickstoffatmosphäre unter Rühren auf 90 °C erhitzt. Anschließend wird die in Tabelle 1 angegebene Menge an Katalysator als 0,1%ige Lösung in Toluol zugegeben. Das Reaktionsgemisch wird in einer Stickstoffatmosphäre unter Rühren bis zum Erreichen des in der folgenden Tabelle angegebenen NCO-Gehalts auf 95 °C erhitzt. Bei der in der Tabelle angegebenen Temperatur wird dem Reaktionsgemisch der Stopper zugesetzt. Danach wird 1 bis 1 ½ Stunden bei 100 °C nachgerührt. Der angegebene NCO-Gehalt wird 24 Stunden nach beendeter Reaktion bestimmt **(A-Versuche).**
2. Eine Teilmenge der so erhaltenen Carbodiimid-modifizierten Isocyanate wird zur Beurteilung seiner Lagerstabilität in einer dicht verschlossenen Aluminiumflasche für drei Tage bei 100 °C in einem Trockenschrank verwahrt, bevor NCO-Gehalt und Viskosität erneut bestimmt werden **(B-Versuche).**

**Tabelle 1: Herstellung CD/UI-modifizierter Isocyanate (A-Versuche) und Lagertests (B-Versuche)**

| | **Einheit** | **Beispiel A1 (Vergleich)** | **Beispiel A2 (erfindungsgemäß)** |
|---|---|---|---|
| Desmodur 44M® | [g] | 1000 | 1000 |
| Katalysator | [g] | 0,00400 | 0,00427 |
| TFMSEE | [g] | 0,050 | 0 |
| Diphenylsilan | [g] | 0 | 0,1554 |
| Reaktionstemperatur | [°C] | 90 | 90-100 |
| Stopperzugabe bei | [NCO-%] | 29,7 | 30,2 |
| Stopperzugabe bei | [°C] | 90 | 60 |
| Nachreaktion bei 100 °C | [h] | 1,5 | 1,0 |
| Aussehen bei Raumtemp. | [-] | klar | klar |
| NCO-Gehalt | [Massen-%] | 29,45 | 29,77 |
| Viskosität bei 25°C | [mPa·s] | 33 | 27 |
| Hazen-Farbzahl | [-/-] | 420 | 120 **B2** |

| | **Einheit** | **Beispiel B1 (Vergleich)** | **Beispiel (erfindungsgemäß**) |
|---|---|---|---|
| NCO-Gehalt | [Massen-%] | 27,31 | 28,21 |
| NCO-Abnahme | [Prozentpunkte] | 2,14 | 1,56 |
| Viskosität bei 25 °C | [mPa·s] | 73 | 62 |
| Viskositäts-Zunahme | [mPa·s] | 40 | 35 |

Das CD-modifzierte Isocyanat aus Versuch A2 weist bei einem dem CD-modifzierten Isocyanat aus Versuch A1 vergleichbaren NCO-Gehalt eine deutlich bessere Hazen-Farbzahl auf. Nach Lagerung ist der NCO-Gehalt des Folgeprodukts von A2 (Produkt aus Versuch B2) signifikant höher als der des Folgeprodukts von A1 (Produkt aus Versuch B1).

In weiteren Versuchen wurden modifizierte Isocyanate wie oben beschrieben hergestellt und Lagertests unterworfen. Die Ergebnisse sind in Tabelle 2 zusammengefasst.

**Tabelle 2: Lagertests (3 d, 100 °C) modifizierter Isocyanate**

| **Beispiel:** | **B11 (Vgl.)** | **B3** | **B22** | **B4** | **B5 (Vgl.)** |
|---|---|---|---|---|---|
| **Stopper:** | **TFMSEE** | **Phenylsilan** | **Diphenylsilan** | **Triphenylsilan** | **Baysilon MH15** |
| **(Versuchsanzahl):** | **(6) MW/Ab w^{a}** | **(3) MW/Abw^{a}** | **(2) MW/Abw^{a}** | **(2) MW/Abw^{a}** | **(1)** |
| Hazen-Farbzahl des frischen Produkts [-] | 372/189 | 153/13 | 150/0 | 199/32 | 200 |
| NCO-Abnahme [Prozentpunkte] | 1,80/0,53 | 2,00/0,21 | 2,70/0,11 | 1,40/0,02 | 1,64 |
| Viskositäts-Zunahme [mPa · s] | 37,0/13,1 | 39,0/7,0 | 57,0/1,0 | 29,0/2,0 | 26,0 |
| Molverhältnis Katalysator : Stopper | 1 : 13-15 | 1 : 15-40 | 1 : 15-30 | 1 : 15-25 | 1000^{b} |
| Anmerkung | c | - | d | - | e |

| | | | | | |
|---|---|---|---|---|---|
| a) MW = Mittelwert; Abw = größte Abweichung vom Mittelwert. b) Angabe abweichend von den übrigen Einträgen in *ppm Phospholinoxid bezogen auf die Gesamtmasse des Reaktionsansatzes.* c) Versuchsreihe enthaltend Versuch B1. d) Versuchsreihe enthaltend Versuch B2. e) Produkt war zweiphasig. | | | | | |

Diese zusätzlichen Beispiele belegen, dass der Zusatz verschiedener Silane Carbodiimidmodifiziertes MDI in ähnlicher Weise stabilisiert wie der aus dem Stand der Technik bekannte TFMSEE, gleichzeitig aber zu niedrigeren Farbzahlen führt. Versuch B5 zeigt, dass mit anderen als den erfindungsgemäßen Silan-Stoppern zwar auch eine gewisse Stabilisierung erreicht werden kann, dies jedoch mit anderen Nachteilen verbunden ist. Die Molverhältnisse *Katalysator : Stopper* können in breitem Rahmen variiert werden.

### II. Herstellung von Präpolymeren:

Die beiden frisch hergestellten modifizierten Isocyanate aus den Versuchen A1 und A2 werden zu (Semi-)Präpolymeren (Präpolymere mit einem höheren Massenanteil an monomerem Isocyanat als an oligomerisiertem Isocyanat - im Rahmen dieser Erfindung werden solche Verbindungen auch als Präpolymere aufgefasst) verarbeitet, indem die modifizierten Isocyanate jeweils mit Irganox 1076 versetzt, unter Stickstoff in einem mit Rückflusskühler, Rührer und Gaseinlass ausgestattetem Dreihalskolben vorgelegt und dann bei 80 °C mit einem deutlichen molaren Unterschuss des Polyols entsprechend der in Tabelle 2 angegebenen Rezeptur umgesetzt werden.

Nach einer Reaktionszeit von 2,0 h ist das Polyol vollständig umgesetzt, und die Reaktionsmischung wird auf Raumtemperatur abgekühlt **(C-Versuche).** Auch an diesen Präpolymeren werden vergleichende Tests zur Beurteilung ihrer Lagerstabilität wie zuvor beschrieben durchgeführt **(D-Versuche),** deren Ergebnisse ebenfalls in Tabelle 3 angegeben sind.

**Tabelle 3: Herstellung von Präpolymeren aus den CD-modifizierten Isocyanaten und Lagertests**

| | **Einheit** | **Beispiel C1 (Vergleich)** | **Beispiel C2 (erfindungsgemäß)** |
|---|---|---|---|
| Produkt aus Beispiel A1 | [Massen-%]^{a} | 92,32 | 0 |
| Produkt aus Beispiel A2 | [Massen-%]^{a} | 0 | 92,32 |
| Irganox 1076 | [Massen-%]^{a} | 0,18 | 0,18 |
| Polyol | [Massen-%]^{a} | 7,5 | 7,5 |
| NCO-Gehalt | [%] | 24,09 | 24,45 |
| Viskosität bei 25 °C | [mPa · s] | 341 | 270 |
| Hazen-Farbzahl | [-/-] | 480 | 150 |

| | **Einheit** | **Beispiel D1 (Vergleich)** | **Beispiel D2 (erfindungsgemäß)** |
|---|---|---|---|
| NCO-Gehalt | [%] | 23,58 | 24,04 |
| NCO-Abnahme | [Prozentpunkte] | 0,51 | 0,41 |

| | | | |
|---|---|---|---|
| a) Bezogen auf die Gesamtmasse des Reaktionsansatzes. | | | |

Das Präpolymer aus Versuch D1 weist bei ansonsten gleicher Zusammensetzung des Reaktionsansatzes und gleichen Reaktionsbedingungen einen höheren NCO-Gehalt, eine geringere Viskosität und eine deutlich niedrigere Hazen-Farbzahl als das Präpolymer aus Versuch C1 auf. Diese Trends bestätigen sich bei Lagerung.

## Patentansprüche

1. Verfahren zur Herstellung Carbodiimid- und/oder Uretonimingruppen aufweisender organischer Isocyanate oder davon abgeleiteter Präpolymere, bei dem
(i) in einem ersten Schritt ein Ausgangsisocyanat, das ein organisches Isocyanat oder ein Gemisch mehrerer organischer Isocyanate ist, mit einem Katalysator K der Formel *cyclo*-C₄H₆P(O)R, wobei der Substituent R für einen gesättigten oder ungesättigten, optional substituierten, organischen Rest, steht, teilweise carbodiimidisiert wird und anschließend
(ii) in einem zweiten Schritt die Carbodiimidisierungsreaktion bei einer Temperatur ≤ 80 °C durch Zugabe eines Stoppers abgestoppt wird,
wobei als Stopper ein organisches Silan S der allgemeinen Formel HₙSiX₄₋ₙ, wobei n eine natürliche Zahl im Bereich von 1 bis 3 ist, wobei X für einen gesättigten oder ungesättigten, optional substituierten, organischen Rest steht, eingesetzt wird.

2. Verfahren gemäß Anspruch 1, bei welchem als Ausgangsisocyanat ein solches mit einer APHA-Hazen-Farbzahl von ≤ 100 eingesetzt wird.

3. Verfahren gemäß einem der vorstehenden Ansprüche, bei welchem das Ausgangsisocyanat ausgewählt ist aus der Gruppe bestehend aus
• Toluylendisocyanat (TDI) und
• Methylendiphenyldiisocyanat als Isomerengemisch in beliebigen Anteilen der 2,2'-, 2,4'- und 4,4'-Isomeren.

4. Verfahren gemäß Anspruch 1 oder 2, bei welchem das Ausgangsisocyanat ausgewählt ist aus der Gruppe bestehend aus
• 4,4'-Methylendiphenyldiisocyanat enthaltend bis zu 4,0 Massen-% 2,2'-Methylendiphenyldiisocyanat, bezogen auf die Gesamtmasse aller Methylendiphenyldiisocyanat-Isomeren und
• Gemischen aus 2,4'-Methylendiphenyldiisocyanat und 4,4'-Methylendiphenyldiisocyanat enthaltend bis zu 4,0 Massen-% 2,2'-Methylendiphenyldiisocyanat, bezogen auf die Gesamtmasse aller Methylendiphenyldiisocyanat-Isomeren.

5. Verfahren gemäß einem der vorstehenden Ansprüche, wobei das Ausgangsisocyanat mindestens 99,0 Massen-%, bezogen auf die Gesamtmasse des Ausgangsisocyanats, an Methylendiphenyldiisocyanat-Isomeren, enthält, wobei der Anteil des 4,4'-Methylendiphenyldiisocyanats, bezogen auf die Gesamtheit aller Methylendiphenyldiisocyanat-Isomeren im Ausgangsisocyanat, mindestens 97,0 % beträgt.

6. Verfahren gemäß Anspruch 1 oder 2, bei welchem das Ausgangsisocyanat ein Gemisch aus Methylendiphenyldiisocyanat-Isomeren und Polymethylenpolyphenylenpolyisocyanat mit einem Gehalt an Methylendiphenyldiisocyanat-Isomeren von insgesamt 15 Massen-% bis < 95 Massen-%, bezogen auf die Gesamtmasse von Methylendiphenyldiisocyanat-Isomeren und Polymethylenpolyphenylenpolyisocyanat, ist.

7. Verfahren gemäß einem der vorstehenden Ansprüche, bei welchem gilt:
X = Phenyl und n = 2 oder 3.

8. Verfahren gemäß einem der vorstehenden Ansprüche, bei welchem der Katalysator K vom Phospholinoxid-Typ eine der folgenden Verbindungen oder ein Gemisch aus beiden ist:

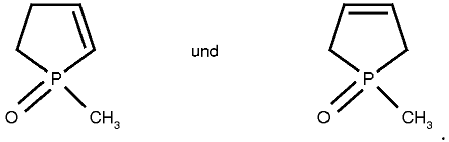

9. Verfahren gemäß einem der vorstehenden Ansprüche, bei welchem die Carbodiimidisierungsreaktion in Schritt (i) im Temperaturbereich von 50 °C bis 150 °C durchgeführt wird.

10. Verfahren gemäß einem der vorstehenden Ansprüche, bei welchem die Carbodiimidisierungsreaktion aus Schritt (i) bei Erreichen eines Carbodiimidisierungsgrades im Bereich von 3,0 % bis 50 % gemäß Schritte (ii) abgestoppt wird.

11. Verfahren gemäß einem der vorstehenden Ansprüche, bei welchem ein Stoffmengenverhältnis n(S)/n(K) von organischem Silan S zu Katalysator K im Bereich von 1 bis 20 verwendet wird.

12. Verfahren gemäß einem der vorstehenden Ansprüche, bei welchem die Reaktionsmischung aus Schritt (i) vor der Durchführung von Schritt (ii) auf eine Temperatur im Bereich von 50 °C bis 70 °C abgekühlt wird.

13. Verfahren gemäß einem der vorstehenden Ansprüche, bei welchem in Schritt (ii) neben dem Silan S kein weiterer Stopper eingesetzt wird.

14. Verfahren gemäß einem Ansprüche 1 bis 12, bei welchem in Schritt (ii) zusätzlich zum Silan S ein weiterer Stopper ausgewählt aus der Gruppe bestehend aus Säuren, Säurechloriden, Sulfonsäureestern und Gemischen aus den vorgenannten Stoffen eingesetzt wird.

15. Verfahren gemäß einem der vorstehenden Ansprüche, bei welchem das in Schritt (ii) erhaltene Carbodiimid- und/oder Uretonimingruppen aufweisende organische Isocyanat mit einem Polyol zu einem Präpolymer umgesetzt wird.

## Claims

1. Process for preparing organic isocyanates having carbodiimide and/or uretonimine groups or prepolymers derived therefrom, wherein
(i) in a first step, a starting isocyanate which is an organic isocyanate or a mixture of a plurality of organic isocyanates is partially carbodiimidized using a catalyst K of the formula *cyclo*-C₄H₆P(O)R, where the substituent R is a saturated or unsaturated, optionally substituted, organic radical, and subsequently
(ii) in a second step, the carbodiimidization reaction is stopped at a temperature of ≤ 80°C by addition of a stopper,
where an organic silane S of the general formula HₙSiX₄₋ₙ, where n is a natural number in the range from 1 to 3 and X is a saturated or unsaturated, optionally substituted, organic radical, is used as stopper.

2. Process according to Claim 1, wherein an isocyanate having an APHA Hazen color number of ≤ 100 is used as starting isocyanate.

3. Process according to either of the preceding claims, wherein the starting isocyanate is selected from the group consisting of
• tolylene diisocyanate (TDI) and
• methylenedi(phenyl isocyanate) as isomer mixture in any proportions of the 2,2', 2,4' and 4,4' isomers.

4. Process according to Claim 1 or 2, wherein the starting isocyanate is selected from the group consisting of
• 4,4'-methylenedi(phenyl isocyanate) containing up to 4.0% by mass of 2,2'-methylenedi(phenyl isocyanate), based on the total mass of all methylenedi(phenyl isocyanate) isomers, and
• mixtures of 2,4'-methylenedi(phenyl isocyanate) and 4,4'-methylenedi(phenyl isocyanate) containing up to 4.0% by mass of 2,2'-methylenedi(phenyl isocyanate), based on the total mass of all methylenedi(phenyl isocyanate) isomers.

5. Process according to any of the preceding claims, wherein the starting isocyanate contains at least 99.0% by mass, based on the total mass of the starting isocyanate, of methylenedi(phenyl isocyanate) isomers, where the proportion of 4,4'-methylenedi(phenyl isocyanate), based on the totality of all methylenedi(phenyl isocyanate) isomers in the starting isocyanate, is at least 97.0%.

6. Process according to Claim 1 or 2, wherein the starting isocyanate is a mixture of methylenedi(phenyl isocyanate) isomers and polymethylenepolyphenylene polyisocyanate having a total content of methylenedi(phenyl isocyanate) isomers of from 15% by mass to < 95% by mass, based on the total mass of methylenedi(phenyl isocyanate) isomers and polymethylenepolyphenylene polyisocyanate.

7. Process according to any of the preceding claims, wherein:
X = phenyl and n = 2 or 3.

8. Process according to any of the preceding claims, wherein the catalyst K of the phospholine oxide type is one of the following compounds or a mixture of the two:

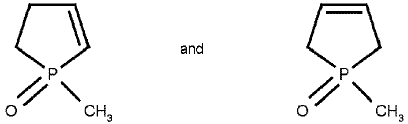

9. Process according to any of the preceding claims, wherein the carbodiimidization reaction in step (i) is carried out in the temperature range from 50°C to 150°C.

10. Process according to any of the preceding claims, wherein the carbodiimidization reaction of step (i) is stopped according to step (ii) on reaching a degree of carbodiimidization in the range from 3.0% to 50%.

11. Process according to any of the preceding claims, wherein a molar ratio n(S)/n(K) of organic silane S to catalyst K in the range from 1 to 20 is used.

12. Process according to any of the preceding claims, wherein the reaction mixture of step (i) is cooled to a temperature in the range from 50°C to 70°C before carrying out step (ii).

13. Process according to any of the preceding claims, wherein no further stopper in addition to the silane S is used in step (ii).

14. Process according to any of Claims 1 to 12, wherein a further stopper selected from the group consisting of acids, acid chlorides, sulfonic esters and mixtures of the abovementioned substances is used in addition to the silane S in step (ii).

15. Process according to any of the preceding claims, wherein the organic isocyanate having carbodiimide and/or uretonimine groups which is obtained in step (ii) is reacted with a polyol to give a prepolymer.

## Revendications

1. Procédé pour la préparation d'isocyanates organiques présentant des groupes carbodiimide et/ou urétonimine ou de prépolymères dérivés de ceux-ci, dans lequel
(i) dans une première étape, on carbodiimidise partiellement un isocyanate de départ, qui est un isocyanate organique ou un mélange de plusieurs isocyanates organiques, avec un catalyseur K de formule cyclo-C₄H₆P(O)R, le substituant R représentant un radical organique saturé ou insaturé, éventuellement substitué, et ensuite
(ii) dans une deuxième étape, on arrête la réaction de carbodiimidisation à une température ≤ 80°C par addition d'un agent d'arrêt,
un silane organique S de formule générale HₙSiX₄₋ₙ, n étant un nombre naturel dans la plage de 1 à 3, X représentant un radical organique saturé ou insaturé, éventuellement substitué, étant utilisé comme agent d'arrêt.

2. Procédé selon la revendication 1, dans lequel on utilise comme isocyanate de départ un isocyanate présentant un indice de couleur APHA-Hazen ≤ 100.

3. Procédé selon l'une quelconque des revendications précédentes, dans lequel l'isocyanate de départ est choisi dans le groupe constitué par
- le diisocyanate de toluylène (TDI) et
- le diisocyanate de méthylènediphényle sous forme de mélange d'isomères en des proportions quelconques des isomères 2,2', 2,4' et 4,4'.

4. Procédé selon la revendication 1 ou 2, dans lequel l'isocyanate de départ est choisi dans le groupe constitué par
- le diisocyanate de 4,4'-méthylènediphényle contenant jusqu'à 4,0% en masse de diisocyanate de 2,2'-méthylènediphényle, par rapport à la masse totale de tous les isomères de diisocyanate de méthylènediphényle et
- les mélanges de diisocyanate de 2,4'-méthylènediphényle et de diisocyanate de 4,4'-méthylènediphényle contenant jusqu'à 4,0% en masse de diisocyanate de 2,2'-méthylènediphényle, par rapport à la masse totale de tous les isomères de diisocyanate de méthylènediphényle.

5. Procédé selon l'une quelconque des revendications précédentes, l'isocyanate de départ contenant au moins 99,0% en masse, par rapport à la masse totale de l'isocyanate de départ, d'isomères de diisocyanate de méthylènediphényle, la proportion du diisocyanate de 4,4'-méthylènediphényle, par rapport à la totalité des isomères de diisocyanate de méthylènediphényle, étant d'au moins 97,0%.

6. Procédé selon la revendication 1 ou 2, dans lequel l'isocyanate de départ est un mélange d'isomères de diisocyanate de méthylènediphényle et de polyisocyanate de polyméthylène-polyphénylène présentant une teneur en isomères de diisocyanate de méthylènediphényle d'au total 15% en masse jusqu'à < 95% en masse, par rapport à la masse totale d'isomères de diisocyanate de méthylènediphényle et de polyisocyanate de polyméthylène-polyphénylène.

7. Procédé selon l'une quelconque des revendications précédentes, dans lequel :
X = phényle et
n = 2 ou 3.

8. Procédé selon l'une quelconque des revendications précédentes, dans lequel le catalyseur K du type oxyde de phosphine est l'un des composés suivants ou un mélange des deux :

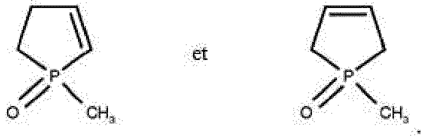

9. Procédé selon l'une quelconque des revendications précédentes, dans lequel la réaction de carbodiimidisation dans l'étape (i) est réalisée dans la plage de température de 50°C à 150°C.

10. Procédé selon l'une quelconque des revendications précédentes, dans lequel la réaction de carbodiimidisation de l'étape (i) est arrêtée selon l'étape (ii) lorsqu'un taux de carbodiimidisation dans la plage de 3,0% à 50% est atteint.

11. Procédé selon l'une quelconque des revendications précédentes, dans lequel on utilise un rapport de quantités n(S)/n(K) de silane organique S au catalyseur K dans la plage de 1 à 20.

12. Procédé selon l'une quelconque des revendications précédentes, dans lequel le mélange réactionnel de l'étape (i) est refroidi avant la réalisation de l'étape (ii) à une température dans la plage de 50°C à 70°C.

13. Procédé selon l'une quelconque des revendications précédentes, dans lequel on n'utilise pas d'autre agent d'arrêt que le silane S dans l'étape (ii) .

14. Procédé selon l'une quelconque des revendications 1 à 12, dans lequel, dans l'étape (ii), on utilise, en plus du silane S, un autre agent d'arrêt choisi dans le groupe constitué par les acides, les chlorures d'acide, les esters d'acide sulfonique et les mélanges des substances susmentionnées.

15. Procédé selon l'une quelconque des revendications précédentes, dans lequel l'isocyanate organique présentant des groupes carbodiimide et/ou des groupes urétonimine obtenu dans l'étape (ii) est transformé avec un polyol en prépolymère.
